# EUROPEAN PATENT SPECIFICATION

(11) **EP 4 594 476 B1**
(45) Date of publication and mention of the grant of the patent: **14.10.2026**
(21) Application number: 23782897.5
(22) Date of filing: 29.09.2023
(51) Int. Cl.: C12N 5/0793, C12N 5/0797, C07K 14/78

(54) **LAMININ CELL CULTURE SUBSTRATE AND PROTOCOL**
ZELLKULTURSUBSTRAT AUS LAMININ UND ZELLKULTUREMETHODE
SUBSTRATE DE CULTURE A BASE DE LAMININE ET METHODE POUR LA CULTURE DE CELLULES

(30) Priority: 29.09.2022 GB 202214291
(43) Date of publication of application: 06.08.2025
(73) Proprietor: Rinri Therapeutics Limited, Sheffield S1 4DP (GB)
(72) Inventor: RIVOLTA, Carlos Marcelo Nicolas, Sheffield South Yorkshire S1 4DP (GB); ABBAS, Leila, Sheffield South Yorkshire S1 4DP (GB)
(74) Representative: Bassil, Nicholas Charles
(86) International application number: PCT/EP2023/077067
(87) International publication number: WO 2024/068934

(56) References cited:
- WO-A1-2016/156831
- WO-A1-2017/072580
- WO-A2-2016/162747
- RODGERS KATHYRN D ET AL: "The laminins in the murine inner ear: developmental transitions and expression in cochlear basement membranes", HEARING RESEARCH, vol. 158, no. 1-2, 1 August 2001 (2001-08-01), NL, pages 39 - 50, XP093112553, ISSN: 0378-5955, DOI: 10.1016/S0378-5955(01)00283-0
- PACCOLA MESQUITA FERNANDA C. ET AL: "Laminin as a Potent Substrate for Large-Scale Expansion of Human Induced Pluripotent Stem Cells in a Closed Cell Expansion System", STEM CELLS INTERNATIONAL, vol. 2019, 22 January 2019 (2019-01-22), US, pages 1 - 9, XP093112600, ISSN: 1687-966X, Retrieved from the Internet <URL:http://downloads.hindawi.com/journals/sci/2019/9704945.xml> [retrieved on 20231215], DOI: 10.1155/2019/9704945
- ANONYMOUS: "STEM CELL CULTURE MATRICES A practical guide", 1 January 2019 (2019-01-01), Sweden, XP093112690, Retrieved from the Internet <URL:https://biolamina.com/wp-content/uploads/2021/06/stem-cell-culture-matrices-practical-guide.pdf> [retrieved on 20231215], DOI: Students time
- AUMAILLEY M ET AL: "A simplified laminin nomenclature", MATRIX BIOLOGY, ELSEVIER, NL, vol. 24, no. 5, 1 August 2005 (2005-08-01), pages 326 - 332, XP025300254, ISSN: 0945-053X, [retrieved on 20050801], DOI: 10.1016/J.MATBIO.2005.05.006
- WEI CHEN ET AL: "Restoration of auditory evoked responses by human ES-cell-derived otic progenitors", NATURE, vol. 490, no. 7419, 12 September 2012 (2012-09-12), pages 278 - 282, XP055503958, DOI: 10.1038/nature11415

## Description

The present invention relates to a method for expanding a population of otic neural progenitor (ONP) cells, and compositions for use in the method.

Deafness is a condition with a high prevalence worldwide, produced primarily by the loss of the sensory hair cells and their associated spiral ganglion neurons (SGNs). Of all the forms of deafness, auditory neuropathy is of particular concern. This condition, defined primarily by damage to the SGNs with relative preservation of the hair cells, is responsible for a substantial proportion of patients with hearing impairment. Although the loss of hair cells can be circumvented partially by a cochlear implant, no routine treatment is available for sensory neuron loss, as poor innervation limits the prospective performance of an implant. Using stem cells to recover the damaged sensory circuitry is a potential therapeutic strategy.

Protocols have been developed to induce differentiation from human embryonic stem cells (hESCs) using signals, such as FGF3 and FGF10, involved in the initial specification of the otic placode (Chen et al. (2012) Nature, 490(7419):278-82. doi: 10.1038/nature11415, cited for reference only). Otic progenitors have also been produced from induced pluripotent stem cells (iPSCs) (Boddy et al. (2020) Stem Cells Int. Article ID 3692937, p1-10). The induced otic progenitors are able to differentiate *in vitro* into hair-cell-like cells and auditory neurons that display expected electrophysiological properties. Moreover, when transplanted into an auditory neuropathy model, otic neuroprogenitors engraft, differentiate and significantly improve auditory-evoked response thresholds. However, the FGF3/10 induction method is inefficient, yielding approximately 20% of the required cell types only.

Laminins are heterotrimeric extracellular matrix molecules that form suprastructural networks in basement membranes and elsewhere. They interact with integrin receptors, playing key roles in modulating programs of cytodifferentiation and maintaining tissue homeostasis in animals. Early studies (E.g. Cosgrove et al. (1997) Hearing Research 105: 159-170) demonstrated an extensive laminin network in both the developing and adult cochlea, primarily associated with the basement membranes but did not address the laminin chain composition of these networks. Laminin isoform composition in cochlear basement membranes was investigated in Rodgers et al. (Rodgers et al. (2001) Hearing Research 158: 39-50), which showed that laminin composition varied according to location and developmental stage in cochlear basement membranes and suggested that laminin 211, laminin 221, laminin 311 and laminin 321 (laminin-2,-4,-6, and-7) may be present in the spiral ganglion from postnatal day 7 (P7) to adulthood in mice, and laminin 211 was observed from P2, the earliest time point.

Laminin (also referred to herein as 'LN') was first isolated from Engelbreth-Holm-Swarm (EHS) mouse sarcoma cells and is a component of Matrigel^{™}, which is commonly used as a substrate for culturing cells. Specific laminins such as murine laminin 111 have been used as a substrate for cell culture. Paccola Mesquita et al. (Paccola Mesquita et al. (2019) Stem Cells International Article ID 9704945) demonstrated expansion of iPSCs in a closed cell culture system using laminin 521 as a substrate.

The number of high-quality cells required for engineering an adult human-sized bioartificial organ is greater than one billion. An implant of ONPs to treat sensory neuron loss may require fewer cells, probably in the 10⁵ range per dose. But the manufacture of suitable batches to treat multiple patients will require the generation of tens of millions of relevant cells. Methods and substrates are required that permit expansion of ONPs whilst retaining their neural potential. For otic progenitors to be used in a clinical setting, methods and compositions are required that enable the expansion of ONPs in a chemically defined, and animal origin free cell culture system.

According to a first aspect of the invention there is provided an otic neural progenitor (ONP) cell culture substrate (also referred to as substrate herein) comprising a protein matrix composed of laminin 211, laminin 111 and laminin 521, wherein the amounts of laminin 211, the laminin 111 and the laminin 521 in the otic neural progenitor (ONP) cell culture substrate are defined by a ratio X:Y:Z of (laminin 211):(laminin 111):(laminin 521). X can be from about 1 to about 10. Y can be from about 1 to about 5. Z can be from about 1 to about 5.

The invention also provides a method for expanding a population of otic neural progenitor (ONP) cells, the method comprising (a) culturing the cells on a first cell culture substrate composed of laminin 211, laminin 111 and laminin 521 for one passage; and subsequently (b) culturing the cells on a second cell culture substrate composed of laminin 211, laminin 111 and laminin 521 for a further passage, wherein the amount of laminin 211 in the second cell culture substrate is increased relative to the amounts of laminin 111 and laminin 521 in the first cell culture substrate. In optional step (c) the cells are cultured on a third cell culture substrate composed of laminin 211, laminin 111 and laminin 521 wherein the amount of laminin 211 in the third cell culture substrate is increased relative to the amounts of laminin 111 and laminin 521 in the second cell culture substrate.

Laminin ratio refers to the ratio of LN211:LN521:LN111 unless otherwise stated. Where the ratio is provided in terms of X, Y and Z, X is LN211, Y is LN521 and Z is LN111 unless otherwise stated.

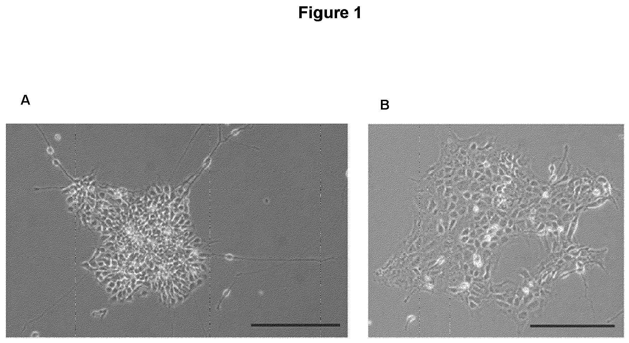

Figure 1 shows colonies of ONPs (A) and OEPs (B) showing their characteristic morphologies. Figure 1 (A) shows ONPs. Figure 1 (B) shows OEPs. The scale bar represents 200 µm.

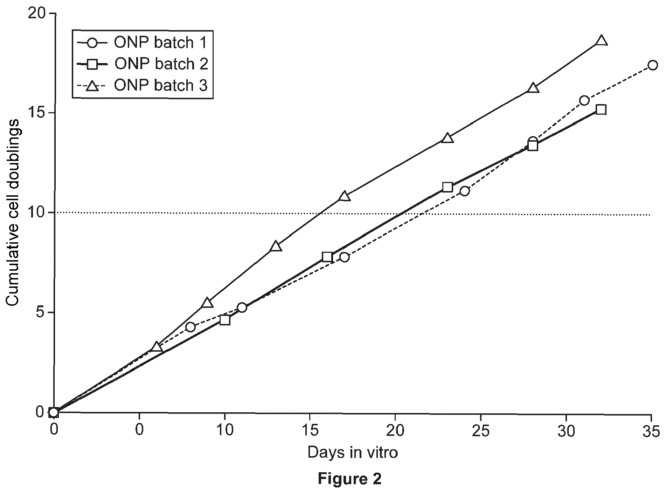

Figure 2 shows cumulative cell doublings over a period of 35 days for three separate batches of ONPs. ONPs remain proliferative over successive passages, resulting in about 15 cumulative cell doublings in 30 days. Cumulative population cell doublings of three different batches of ONPs, expanded on the matrices shown on table 2. Each point of the plots represents a passage. ONP batch 1 is indicated by a circle. ONP batch 2 is indicated by a square. ONP batch 3 is indicated by a triangle.

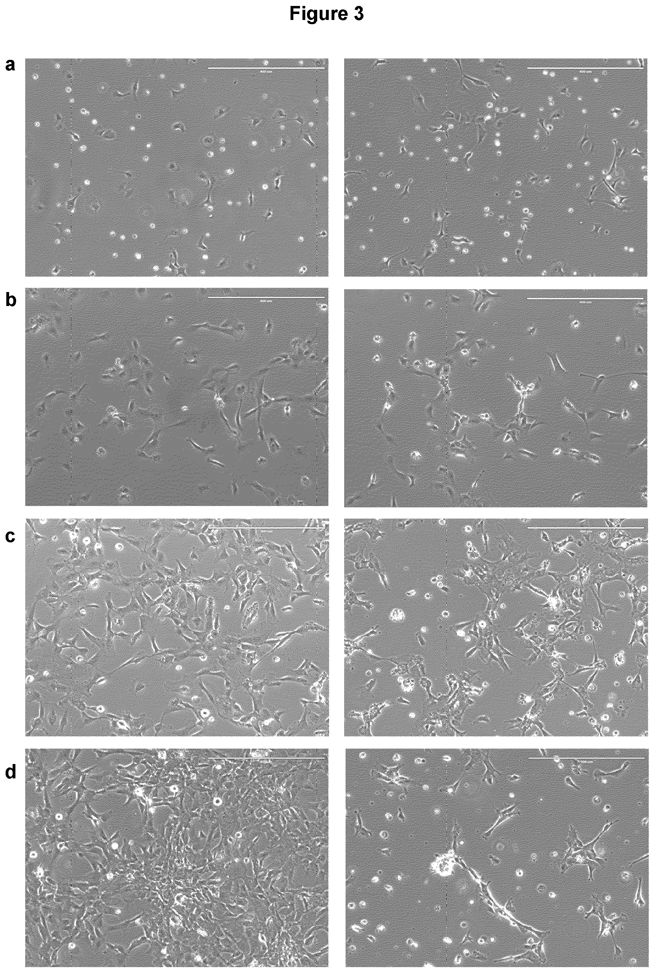

Figure 3 shows that plating the same ONP population, directly after sorting, onto a laminin mixture containing a higher proportion of LN211, affects attachment and morphology. For Figure 3 a to d, the panel on the left hand side (LHS) shows ONP cells plated directly after sorting onto a substrate with a laminin ratio 1:1:1, and the panel on the right hand side (RHS) shows ONP cells plated directly after sorting onto a substrate with a laminin ratio 6:1:1 (i.e. the proportion of LN211 is increased from one third (1/3) to three quarters (3/4)). Figure 3 a shows ONP cells 2 days after plating. Figure 3 b shows ONP cells 4 days after plating. Figure 3 c shows ONP cells 6 days after plating. Figure 3 d shows ONP cells 7 days after plating. The scale bar represents 400 µm.

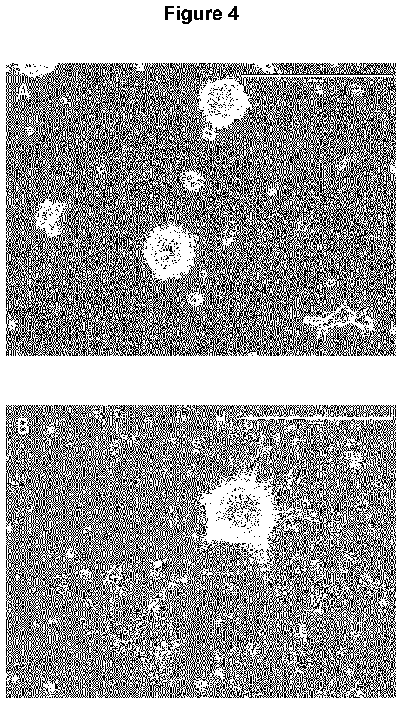

Figure 4 shows that plating ONPs directly after sorting onto a laminin mixture containing a higher proportion of LN211 affects attachment and morphology. Figure 4 A and Figure 4 B show ONP cells plated directly after sorting onto a substrate with a mixture of LN211:LN521:LN111 with a laminin ratio 6:1:1. Figure 4 A shows the cells 5 days post-seeding. Figure 4 B shows the cells 8 days post-seeding. The scale bar represents 400 µm.

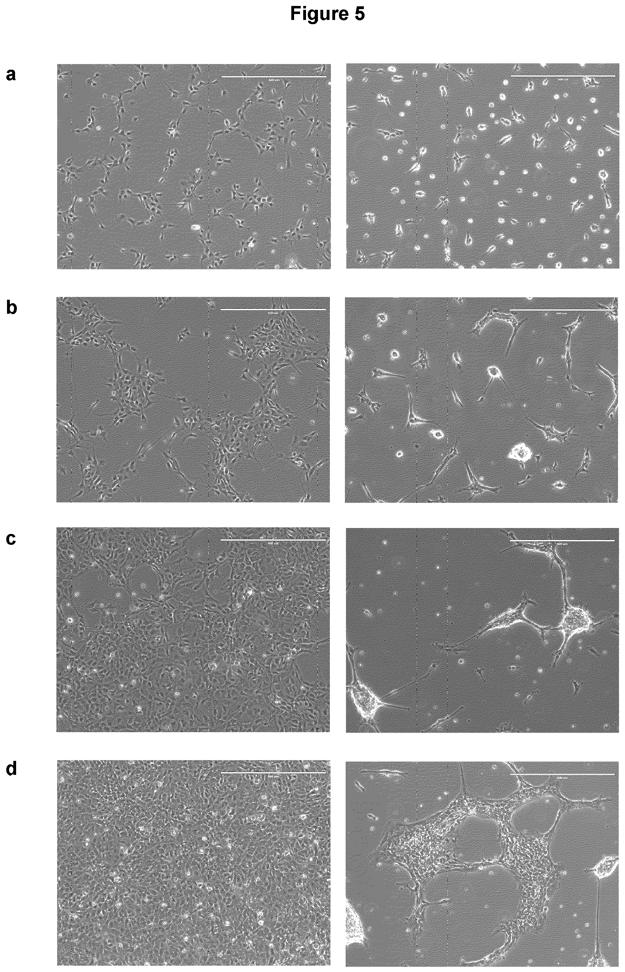

Figure 5 shows plating expanding ONPs on a 6:1:1 substrate onto a 100% LN211 matrix affects cell morphology and attachment. Figure 5 shows expanding ONPs on a substrate with a laminin ratio 6:1:1 after being transferred either to the same matrix (Left Hand Side (LHS) panel), or onto a 100% LN211 matrix (Right Hand Size (RHS) panel). Figures 5 a-d illustrate the difference in ONPS after increasing time periods. Figure 5a) shows the cells 6 hours after plating; Figure 5b) shows the cells 1 day after plating; Figure 5c) shows the cells 3 days after plating; Figure 5d) shows the cells 4 days after plating. The scale bar represents 400 µm.

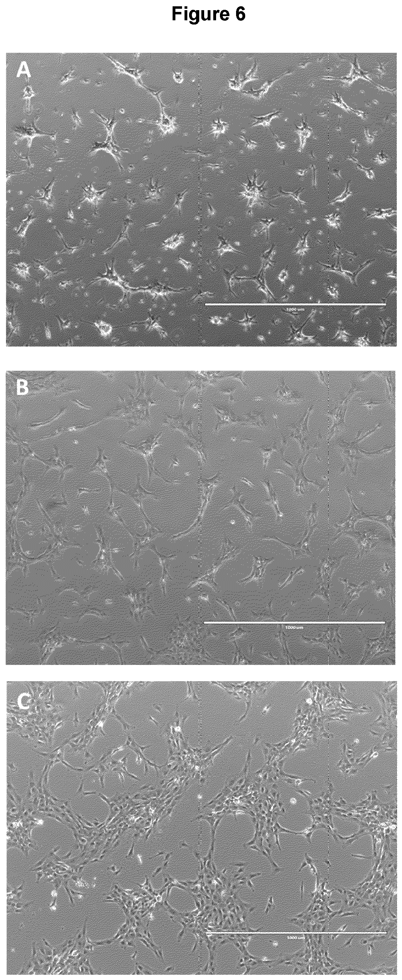

Figure 6 shows that cell morphology and adhesion defects which result from plating expanding ONPs on 100% LN211 can be reversed by plating the cells onto substrate with a laminin ratio 6:1:1. Figure 6 A shows ONPs after 1 day post-plating on 100% LN211. Figure 6 B shows these cells after being passaged a week later onto 100% LN211 (B). Figure 6 C shows these cells after being passaged a week later onto substrate with a laminin ratio 6:1:1 (C). Returning the cells to the 6:1:1 ratio mix restores attachment and cell morphology. The scale bar represents 1000 µm.

The invention provides substrates and methods that can be used in processes that comply with Good Manufacturing Practice (GMP), the minimum standard that a medicines manufacturer must meet in their production processes for certification by the European Medicines Agency and equivalent regulatory agencies. The inventors have surprisingly found that expansion of ONPs can be improved by varying the combination of isoforms of laminin used as a substrate during the expansion. LN211 is a laminin present during development of the cochlea. Expansion can be achieved by increasing the proportion of LN211 present in the substrate stepwise. When the laminin mixture is replaced fully with 100% LN211 expansion can also be compromised. Expansion is more efficient when a combination of LN211, LN511 and LN111 are used in the substrate. Expansion is most efficient when the proportion of LN211 is increased stepwise over a small number of passages (e.g. over 2 to 4 passages, such as 3 passages). ONPs cultured on the substrates described herein comprising LN211, LN511 and LN111 and cultured using the expansion methods described herein remain proliferative. ONPs cultured on the substrates described herein comprising LN211, LN511 and LN111 and cultured using the expansion methods described herein retain good morphology and neural potential.

Laminin comprises three disulphide-linked polypeptides, designated α, β and γ. Each of the polypeptides is found in a number of isoforms, designated by a number following the relevant Greek letter , e.g. α2, β1 and γ1. Standard laminin nomenclature now refers to laminins by their isoform composition, referring to numbers only. Thus a laminin comprising α2, β1 and γ1 is laminin 211 (Aumailley (2013) Cell Adhesion & Migration 7, 48-55), also referred to herein as LN211. Table 1 provides a list of laminins with their composition and corresponding alternative names that have been used in the literature.

**Table 1. Laminin types.**

| **New nomenclature** | **Chain composition** | **Old nomenclature** | **Old synonyms** |
|---|---|---|---|
| Laminin-111 (LN111) | α1β1γ1 | Laminin-1 | EHS laminin |
| Laminin-211 (LN211) | α2β1γ1 | Laminin-2 | Merosin |
| Laminin-121 (LN121) | α1β2γ1 | Laminin-3 | S-laminin |
| Laminin-221 (LN221) | α2β2γ1 | Laminin-4 | S-merosin |
| Laminin-332 (LN322) / Laminin-3A32 (LN3A32) | α3Aβ3γ2 | Laminin-5 / Laminin-5A | Kalinin, epiligrin, nicein, ladsin |
| Laminin-3B32 (LN3B32) | α3Bβ3γ2 | Laminin-5B | |
| Laminin-311 (LN311) / Laminin-3A11 (LN3A11) | α3Aβ1γ1 | Laminin-6 / Laminin-6A | K-laminin |
| Laminin-321 (LN321) / Laminin-3A21 (LN3A21) | α3Aβ2γ1 | Laminin-7 / Laminin-7A | KS-laminin |
| Laminin-411 (LN411) | α4β1γ1 | Laminin-8 | |
| Laminin-421 (LN421) | α4β2γ1 | Laminin-9 | |
| Laminin-511 (LN511) | α5β1γ1 | Laminin-10 | Drosophila-like laminin |
| Laminin-521 (LN521) | α5β2γ1 | Laminin-11 | |
| Laminin-213 (LN213) | α2β1γ3 | Laminin-12 | |
| Laminin-423 (LN423) | α4β2γ3 | Laminin-14 | |
| Laminin-522 (LN522) | α5β2γ2 | | |
| Laminin-523 (LN523) | α5β2γ3 | Laminin-15 | |

The laminin used in substrates and methods provided herein may be recombinant. That is, the laminin may be a recombinant protein, e.g. protein that is overexpressed and purified using techniques known in the art. The laminin is preferably human. Laminin can be purchased, e.g. from BioLamina (Human recombinant laminin), or prepared using standard methods in the art. The amount of laminin in a cell culture substrate is typically measured in micrograms per unit area (e.g. cm²). The total concentration of the laminin should be from 0.5 to 2.0 µg/cm², preferably about 1µg/cm². The total concentration of laminin is the sum of all the laminin isoform concentrations combined.

Otic progenitor cells (also described as otic progenitors herein) are cells which can further differentiate either into hair cells (otic epithelial progenitors, OEPs) or auditory neuronal cells (otic neuronal progenitors, ONPs). OEPs and ONPS each have a characteristic morphology which can be used to distinguish them from each other. OEPs are larger than ONPs with a typical size greater than about 12µm. OEPs show well defined cell-cell junctions. OEPs form epithelioid islands. ONP cells are typically smaller than OEPs with a typical size range about 8µm to about 15µm. ONPs present cytoplasmic projections. ONPs form loosely associated colonies. ONP cells have denser chromatin than OEP cells. ONPs can be identified by morphology (Figure 1). The otic placode is the primordium of the hearing organ. The state of an otic progenitor can be defined by the expression of markers associated with the developing early otic placode. Suitable markers include SOX2, FOXG1, PAX2, PAX8, SSEA1 (also known as CD15 or Lewis x), GD3, TRA-2-49 (alkaline phosphatase), SSEA4, GD2 and CD141. Otic progenitors can express two or more of SOX2, FOXG1, PAX2, and PAX8, such as SOX2 and PAX8, FOXG1 and PAX8, or PAX2 and PAX8. Human otic progenitor cells can be identified as cells which have at least two cell surface markers selected from SSEA1 (also known as CD15 or Lewis x), GD3, TRA-2-49 (alkaline phosphatase), SSEA4, GD2 and CD141. The markers can be quantified e.g. using fluorescence or by qPCR. Suitable methods of preparing such cells are described in WO 2016/156831 and WO 2018/051092. ONP cells may be human ONP cells. An ONP batch typically expresses one or more/two or more markers selected from a group including: SSEA1, ITGA4. An ONP cell with neural potential may express one or more/two or more markers selected from a group including: SSEA1 and GD2. During the expansion of ONPs surface markers may be downregulated, (e.g. SSEA1). Other surface markers may be upregulated (e.g. ITGA4). ONP generation refers to the production of ONP cells from pluripotent cells by differentiation. ONP expansion refers to increasing the number of ONP cells which have the capacity to become auditory neuronal cells, without differentiating the cells into auditory neuronal cells.

Prior to expansion, e.g. during ONP generation, cells (ONP cells and/or differentiating cells) are typically cultured on a substrate comprising laminin 521 (LN521) and laminin 111 (LN111), at a ratio Y:Z LN521:LN111 e.g. where Y=about 1 and Z= about 1. During expansion, the laminin composition of the substrate is progressively changed from that used during ONP generation, to a mixture that includes LN211, so that the laminins LN211, LN521 and LN111 are present in the ratio X:Y:Z LN211:LN521:LN111. X can be from about 1 to about 10. X can be 2-6, 3-6, 4-6, 5-6. X can be 1, 2, 3, 4, 5, 6, 7, 8, 9 or 10. Y can be from about 1 to about 5. Y can be 1-3 or 1-2. Y can be 1, 2, 3, 4 or 5, e.g. 1. Z can be from about 1 to about 5. Z can be 1, 2, 3, 4 or 5, e.g. 1.

In the first passage of expansion LN211 is typically included in the substrate. The laminin ratio is typically changed from Y:Z, where Y=about 1 and Z= about 1, to X:Y:Z, where X = about 1, Y=about 1 and Z= about 1 (such as 1:1:1 LN211:LN521:LN111). Thus the otic neural progenitor (ONP) cell culture substrate used to seed the cells in the first culturing step of the expansion (passage 0 (p0)) typically comprises a protein matrix comprising human laminin 211, human laminin 521 and human laminin 111, wherein the amounts of laminin 211, the laminin 521 and the laminin 111 in the otic neural progenitor (ONP) cell culture substrate are defined by a ratio X:Y:Z of (laminin 211):(laminin 521):(laminin 111) and where X = about 1, Y=about 1 and Z= about 1. The ratio X:Y:Z of the otic neural progenitor (ONP) cell culture substrate in the following culturing step (E.g. second step, passage 1 (p1)) is typically X = about 2, Y=about 1 and Z= about 1. The ratio X:Y:Z of the otic neural progenitor (ONP) cell culture substrate in subsequent culturing steps (e.g. third step (p2), fourth step (p3) and so on) is typically X = about 6, Y=about 1 and Z= about 1 (e.g. 6:1:1). The otic neural progenitor (ONP) cell culture substrate can have the ratio X:Y:Z where X is 1,2,3,4,5 or 6; Y is about 1; and Z is about 1. The otic neural progenitor (ONP) cell culture substrate can have the ratio X:Y:Z where X from about 2 to about 6; Y is about 1; and Z is about 1.

In the method for expanding a population of otic neural progenitor (ONP) cells, the method can comprise a step of culturing the cells on a first cell culture substrate composed of laminin 211, laminin 111 and laminin 521 in the ratio X1:Y:Z of (laminin 211):(laminin 521):(laminin 111). The method can further comprise a step of culturing the cells on a second cell culture substrate composed of laminin 211, laminin 111 and laminin 521 in the ratio X2:Y:Z of (laminin 211):(laminin 521):(laminin 111) for a further passage, wherein X2 is greater than X1. Thus the amount of laminin 211 in the second cell culture substrate is increased relative to the amounts of laminin 111 and laminin 521 in the first cell culture substrate. The method may further comprise a step of culturing the cells on a third cell culture substrate composed of laminin 211, laminin 111 and laminin 521 in the ratio X3:Y:Z of (laminin 211):(laminin 521):(laminin 111) for a further passage wherein X3 is greater than X2. Thus the amount of laminin 211 in the third cell culture substrate is increased relative to the amounts of laminin 111 and laminin 521 in the second cell culture substrate.

Any of the cell culture substrates described herein can be used in the methods for expanding a population of otic neural progenitor cells.

The expansion method may include a step of culturing the ONP cells on a cell culture substrate consisting of laminin 111 and laminin 521 for one passage prior to culturing on a substrate comprising LN211, optionally wherein the laminin 111 and the laminin 521 are human laminin 111 and human laminin 521. Thus the method may further comprise an initial step before step (a) of culturing the ONP cells on a cell culture substrate consisting of laminin 111 and laminin 521 for one passage and subsequently transferring the cells to the cell culture substrate of step (a). In this step the cells typically reach 80%-90% confluence (such as 90% confluence), e.g. after 3 to 10 days of culturing.

In the culturing methods described herein the ONP cells are typically seeded so that they start at about 10% confluence, such as about 20,000-40,000 cells/cm².

The terms passage and culturing step are used interchangeably herein. The time between each passage/culturing step is typically about 1 week, e.g. from 3 to 10 days, such as from 5 to 9 days. The cells can be cultured for about 1 week or until they reach confluence before performing the next culturing step. The cells are typically cultured at 37°C. The cells are cultured in a 5% CO₂ environment. The cells are typically passaged when the cells are 80%-100% confluent, preferably when the cells are 80%-90% confluent, e.g. at about 90% confluence. Passage and culturing are terms known in the art. When the cells are passaged they are typically harvested from the culture vessel. After removal of the liquid media, a cell disassociation buffer solution (e.g. containing trypsin) is used to detach the cells from each other and the vessel. The cells are washed and centrifuged (e.g. 100 g - 200 g for 5-10 minutes) before being suspended in fresh medium for counting, and seeding into a vessel with a laminin substrate described herein for culturing (re-culturing).

The product of the method for expanding a population of otic neural progenitor (ONP) cells is an expanded population of ONP cells, wherein the ONP cells have good morphology and/or neural potential. The population may be in the same vessel, or may be split between two or more vessels. Population doubling time and cumulative population doublings were calculated using the number of cells initially seeded, the number of cells lifted and the length of time between passaging/culturing steps. For example, the population may undergo 5 cumulative population doublings in 10 days (a week) (Figure 2). The cumulative population doublings can be from 2 to 4, or 2 to 3, such as about 3. Preferably the cumulative population doubling is greater than 2, e.g. 2-4. The population doubling time can be less than 50 hours, e.g. 35-45 hours. The population doubling time can be about 38 hours.

The cell culture medium added to the cells on the cell culture substrate may comprise at least one growth factor. The cell culture medium may comprise at least one growth factor selected from the group of an FGF, an EGF and an IGF (such as IGF-I and IGF-2, e.g. IGF-1). The cell culture medium can comprise FGF (such as bFGF), EGF and IGF-1. The growth factors are preferably recombinant growth factors. The concentration of each growth factor in the medium, if present, is typically 10-30 ng/ml, e.g. 20 ng/ml. The growth factors may be human growth factors. The cell culture medium may comprise Dulbecco's Modified Eagle Medium (DMEM). The cell culture medium may comprise L-glutamine, glucose, sodium pyruvate, and sodium bicarbonate (e.g. DMEM comprising 4 mM L-glutamine, 4500 mg/L glucose, 1 mM sodium pyruvate, and 1500 mg/L sodium bicarbonate). The cell culture medium may comprise B27 neuronal culture supplement (e.g. B27 supplement, Thermofisher, Cat. No. 17504044).

Preferred features for the second and subsequent aspects of the invention are as for the first aspect *mutatis mutandis.*

### Embodiments

In the following embodiments of the cell substrate composition and methods described herein are listed.

Embodiment 1. An otic neural progenitor (ONP) cell culture substrate comprising a protein matrix composed of laminin 211, laminin 521 and laminin 111, wherein the amounts of laminin 211, the laminin 521 and the laminin 111 in the otic neural progenitor (ONP) cell culture substrate are defined by a ratio X:Y:Z of (laminin 211):(laminin 521):(laminin 111) in which X is from about 1 to about 10; Y is from about 1 to about 5; and Z is from about 1 to about 5.

Embodiment 2. The otic neural progenitor (ONP) cell culture substrate of embodiment 1, wherein X is from about 2 to about 6; Y is about 1; and Z is about 1.

Embodiment 3. The otic neural progenitor (ONP) cell culture substrate of embodiment 1 or embodiment 2, wherein the laminin 211, laminin 111 and laminin 521 are human laminin 221, human laminin 111 and human laminin 521.

Embodiment 4. The otic neural progenitor (ONP) cell culture substrate of any one of embodiments 1-3, wherein the laminin 211, laminin 111 and laminin 521 are recombinant.

Embodiment 5. A method for expanding a population of otic neural progenitor (ONP) cells, the method comprising: (a) culturing the cells on a first cell culture substrate composed of laminin 211, laminin 111 and laminin 521 for one passage; and subsequently (b) re-culturing the cells on a second cell culture substrate composed of laminin 211, laminin 111 and laminin 521 for a further passage, wherein the amount of laminin 211 in the second cell culture substrate used in step (b) is increased relative to the amounts of laminin 111 and laminin 521 in the first cell culture substrate used in step (a); optionally (c) repeating step (b) wherein the cells are cultured on a third cell culture substrate composed of laminin 211, laminin 111 and laminin 521 and wherein the amount of laminin 211 in the third cell culture substrate used in step (c) is increased relative to the amounts of laminin 111 and laminin 521 in the second cell culture substrate used in step (b).

Embodiment 6. A method as described in embodiment 5, wherein the amounts of laminin 211, the laminin 111 and the laminin 521 in the cell culture substrate in step (a) are defined by a ratio X:Y:Z of (laminin 211):(laminin 111):(laminin 521) in which X is from about 1 to about 10; Y is from about 1 to about 5; and Z is from about 1 to about 5.

Embodiment 7. A method as described in embodiments 5 or 6, wherein the laminin 211, laminin 111 and laminin 521 are (i) human laminin 221, human laminin 111 and human laminin 521 and/or (ii) recombinant.

Embodiment 8. The method as described in any one of embodiments 5 to 7, wherein the ONP cells are human cells.

Embodiment 9. The method as described in any one of embodiments 5 to 8, wherein the method further comprises an initial step before step (a) of culturing the ONP cells on a cell culture substrate consisting of laminin 111 and laminin 521 for one passage and subsequently transferring the cells to the cell culture substrate of step (a), optionally wherein the laminin 111 and the laminin 521 are human laminin 111 and human laminin 521.

Embodiment 10. The method as described in any one of embodiments to 8, wherein a cell culture medium is added to the cells on the cell culture substrate comprising at least one growth factor selected from the group of FGF, EGF and IGF.

### EXAMPLES

### Example 1. Preparation of ONPs

Differentiation of ONPs from hESCs and iPSCs generation of otic progenitors was achieved by plating iPSCs in Dulbecco's Modified Eagle Medium: Ham's F12 (DMEM/F12) supplemented with 1x N2 and B27 (abbreviated as DFNB) (all Life Technologies, UK), FGF3 and FGF10 (both 50 ng/ml) (both R&D Systems, UK) onto laminin-coated tissue culture plastic (laminin ratio 1:1 LN521:LN111). The Wnt inhibitor IWR-1 (10µM) was also present. The cells were cultured for 8 days. At day 9, media was replaced with DFNB supplemented with FGF3 and FGF10 (both at 25 ng/ml) and the Wnt activator BIO (2mM). Throughout the differentiation, cells were plated onto laminin-coated tissue culture plastic (laminin ratio 1:1 LN521:LN111). Human recombinant laminin was purchased from BioLaminin (LN521-02; LLN111-02; LN211-02). Cells can be further differentiated along hair cell or auditory neuronal lineages, cultures were manually purified by removing cells lacking relevant characteristic progenitor morphologies, enriching specifically for either otic epithelial progenitor (OEP) or otic neuronal progenitor (ONP) phenotypes.

### Example 2: Identification of ONPs and OEPs

ONPs and OEPs can be differentiated from hESCs and iPSCs e.g. according to Example 1 and as described in Chen (2012), cited for reference only, and Boddy (2020) from undifferentiated colonies plated as a monolayer on laminin coated flasks. The differentiation process induces two morphologically distinct types of otic colonies (Figure 1). Otic epithelial progenitor (OEP) cells show a flat phenotype, with large cytoplasm and formed epithelioid islands. Otic epithelial progenitor (OEP) cells show well-defined cell-cell junctions. Otic neural progenitor (ONP) cells are smaller than OEPs, with denser chromatin and present cytoplasmic projections.

Otic neural progenitors can be identified by quantitative microscopy, based on the co-expression of otic markers PAX8, PAX2, FOXG1, and SOX2. Cells previously fixed for 15 min at room temperature in Phosphate-Buffered Saline (PBS) with 4% paraformaldehyde are blocked with 0.1% Triton-X, 5% donkey serum, and 1% bovine serum albumin in PBS. The following primary antibodies can be used: SOX2 (1 : 100, Millipore), FOXG1, PAX2, (all 1 : 100, Abcam UK), PAX8 (1 : 100, Santa Cruz), POU4F1 (BRN3A, 1 : 100, Chemicon), and B-tubulin III (1 : 100, Sigma). Suitable secondary antibodies include anti-mouse, anti-goat, or anti-rabbit Alexa Fluor 488 and 568 (Molecular Probes, Life Technologies, UK). Nuclei can be counterstained with 4',6-diamidino-2- phenylindole (DAPI) (Sigma). Cells can be imaged either on an EVOS FL Cell Imaging System or using the IN Cell Analyzer 2000 system platforms (GE Healthcare). Quantitative immunofluorescence can be performed on the IN Cell Analyzer using the Developer Toolbox. Cells can be exposed to e.g. PAX8 antibodies in combination with those raised against one of the other markers (PAX2, SOX2, or FOXG1) to identify co-expression in individual cells. Highly expressing cells can be defined as those having fluorescence intensities above the 75th percentile for each antibody independently. Populations of cells that are highly positive for two markers (co-expression) can then be identified as otic progenitors.

### Example 3. Purification of ONPs

ONPs can be selected manually. ONPs can be purified using FACS using the markers referred to herein and methods known to the skilled person e.g. by Fluorescent Automated Cell Sorting using SSEA1 and CD141 antibodies using the methods described in WO2018/051092.

After sorting ONP cells are typically cultured on a substrate comprising laminin 521 (LN521) and laminin 111 (LN111), e.g. at a ratio 1:1 LN521:LN111. Between eight to ten days after sorting, the plate/flask where the ONPs were initially seeded reaches ~80-90% confluency.

### Example 4: Transitioning onto different laminin matrices

ONPs purified by cell sorting as taught, for instance in WO 2018/051092 by using SSEA1 as a cell surface marker, were cultured from p0 (i.e. immediately post-sorting) up to p6, at which point they were frozen. Passaging was mostly carried out when the cells were around 90% confluent. Cells were lifted with 0.5x TrypLE Select. Over the first few expansion cycles, the cells were transitioned towards growth on a matrix containing a higher proportion of LN211. At p1, the ratio was increased from 1:1:1 to 2:1:1 LN 211:521:111; at p2, this was raised to 6:1:1 and this was used from thereon (Table 2 and Fig. 2).

**Table 2. Combination of laminin matrices used for the expansion of ONPs.**

| **Expansion stage** | **Laminin ratio (composition)** |
|---|---|
| Generation (pre-sorting) | 1:1 (LN521:LN111) |
| Post-sorting (p0) | 1:1:1 (LN211:LN521:LN111) |
| P1 | 2:1:1 (LN211:LN521:LN111) |
| P2 | 6:1:1 (LN211:LN521:LN111) |
| P3 | 6:1:1 (LN211:LN521:LN111) |
| P4 | 6:1:1 (LN211:LN521:LN111) |
| P5+ | 6:1:1 (LN211:LN521:LN111) |

Figure 2 shows cumulative population cell doublings of three different batches of ONPs, expanded on the matrices shown on table 2. Each point of the plots represents a passage. These growth curves show that ONPs can remain proliferative using the matrix composition described here.

Transitioning the ONPs onto a suitable laminin composition to support efficient expansion is critical. LN211 has to be introduced in a stepwise fashion. It is used in combination with other laminins. When ONPs are plated onto a 6:1:1 mixture immediately after sorting, cells initially attach. However they do not spread and colonies grow upwards into three-dimensional spheres (Fig. 3 and 4). Population doubling time and cumulative population doublings were calculated using the number of cells initially seeded, the number of cells lifted and the length of time between cycles. Population doublings and cell yields are substantially reduced, compared to the 1:1:1 mixture (Table 3).

**Table 3. Impact of matrix composition on cell growth parameters when transferred directly, post-sorting, onto a 6:1:1 mixture**

| **Cells** | **LN Ratio** | **Population Doublings** | **Cells harvested** |
|---|---|---|---|
| ONP sorted-p0 | 1:1:1 | 2.97 | 944,000 |
| ONP sorted-p0 | 6:1:1 | 1.9 | 448,000 |

The impact of transitioning expanding ONPs (p4) to 100% LN211 was explored with cells that have been on the 6:1:1 mix. However, cells struggled to attach and colonies displayed a 'spiky' morphology (Fig. 5). Cells on the 100% LN211 matrix underwent a reduced number of population doublings (Table 4 and 5), and displayed a longer population doubling time (Table 5). Expansion was more efficient on a substrate with a laminin ratio 6:1:1.

**Table 4. Impact of matrix composition on cell growth parameters when expanding ONPs on a 6:1:1 mixture are transferred onto a pure, 211 only substrate.**

| **Cells** | **LN Ratio** | **Population Doublings** | **Cells harvested** |
|---|---|---|---|
| ONP p4 | 6:1:1 | 2.64 | 3,770,000 |
| ONP p4 | LN211 only | 1.34 | 1,520,000 |

**Table 5. Similar experiment to that shown on table 4, displaying population doublings and population doubling times.**

| **Cells** | **LN Ratio** | **Population Doublings** | **Population doubling time (hr)** |
|---|---|---|---|
| ONP p4 | 6:1:1 | 2.52 | 38.0 |
| ONP p4 | LN211 only | 1.63 | 58.9 |

Subsequently, cells harvested from the 100% LN211 matrix were re-plated either on 100% LN211 or on the 6:1:1 mixture. Cells re-plated on 100% LN211 retained the unusual, spiky morphology while those on the 6:1:1 mixture reverted to the characteristic appearance, suggesting the changes introduced by pure LN211 are reversible (Fig. 6).

### Example 5: Expansion of ONPs in culture

All measurements relate to the volume required for a single T12.5cm² flask (E.g. Corning Falcon vented flask, VWR international catalogue number 353107). ONP cells are typically cultured on a substrate comprising laminin 521 (LN521) and laminin 111 (LN111), e.g. at a ratio 1:1 LN521:LN111. During expansion, the laminin composition of the substrate is progressively changed used during ONP generation, to a mixture that includes LN211 being gradually introduced, e.g. from a 1:1 LN521:LN111 ratio to 1:1:1 LN211:521:111. LN211 is a laminin present during development of the cochlea. This transition progresses from a 1:1:1 LN211:521:111 mixture used to seed the cells immediately after sorting (passage 0 (p0)) to a 2:1:1 LN211:521:111 mixture used from the subsequent split (p0->p1), to a 6:1:1 LN211:521:111 mixture used from the next passage onwards (p1->p2 and beyond).

Laminin 211, laminin 521 and laminin 511 aliquots were defrosted at 4°C for a minimum of 30 minutes. Stock solutions of laminin had a concentration of 100µg/ml. 1 ml of Dulbecco's Phosphate Buffered Saline (DPBS) with Ca2+ and Mg2+ (Cat #D8662, stored at 4°C) was measured into a chilled 50ml tube. The laminin was added to the DPBS. The buffer and the laminin were mixed well and swiftly with a chilled P1000 pipette tip and transferred into the T12.5 flask making sure the flask was horizontal throughout. The flask was tilted gently to ensure even coverage. The flask was incubated overnight at 4°C in a horizontal position.

In the first expansion step (p0->p1) 62.5µl of LN 211, 31.25µl of LN521 and 31.25µl LN111 were added for a 2:1:1 ratio. In the subsequent expansion steps (p1->p2 and beyond) 93.75µl of LN 211, 15.625µl of LN521 and 15.625µl LN111 were added for a 6:1:1 ratio.

### Cell seeding and passaging protocol

A stock solution of 4ml DPBS-/-, 2ml OSCFM and 1ml 0.5x TrypLE Select is warmed overnight. 1x stock is diluted 1:1 with DPBS-/-. The laminin coated flask is rinsed with chilled DPBS +/+, then 2x2ml RT DPBS -/-. 1ml warmed OSCFM medium is added and the flask is placed in the 37°C incubator.

To harvest the cells, old media is removed from the culture vessel and the cells are washed twice with 2ml of DPBS (-/-). A cell disassociation buffer solution (TrypLE Select, ThermoFisher) per T12.5 (0.5ml for a single well from a 12-well plate; 0.25ml for a single well from a 24-well plate) is used to remove the cells. The flask is incubated at 37°C for 3 mins before checking for detachment under an inverted microscope. If the cells do not come off easily the plate can be incubated for up to 5 mins in total, checking periodically for detachment.

To collect the cells, dilute the lysis solution with OSCFM per flask and pipette the suspension up and down. Collect and transfer the cell suspension to a 50ml centrifuge tube. Repeat with a further OSCFM wash any remaining cells from the vessel's surface. Centrifuge at 1000rpm (195 x g) for 5 minutes. Re-suspend the pellet with OSCFM. Cell number can be counted using an Automatic Cell Counter (E.g. Biorad TC20). Use the TC20 cell counter to calculate the cells present. Set the gating histogram from 8-22µm and note down the total and live cell counts. Repeat for the other slide chamber and average the two counts. If there is a >10% discrepancy, repeat the above with another sample of cell suspension and average the most concordant 3 readings.

Make a note of the total number of cells obtained. This value (plus the total number of cells initially seeded is used to determine population doublings - PD). Plate at about 20,000-40,000 cells/cm² (i.e. 250,000-500,000 cells in a T12.5 flask), in a total of 2.5ml OSCFM per flask, allowing for the 1ml of OSCFM already in the pre-warmed flask. A typical split ratio is 1:3 although cells should always be counted for accurate determination of PDs. Place flask back into incubator and incubate at 37°C & 5.0% CO₂. Cells should be fed every other day, by completely replacing the old media with fresh, pre-warmed OSCFM. Cells are passaged once a week or when 100% confluent, whichever is the sooner.

**Table 6. Composition of DFNB Media**

| **Media Component** | **Final Concentration** | **Volume for 40 ml (41 ml)** |
|---|---|---|
| DMEM/F-12 with GlutaMAX^{™} | | 40 ml |
| N2 | 1X | 400µl |
| B27 | 1X | 800µl |

**Table 7. Composition of Otic Stem Cell Full Media (OCSFM)**

| **Media Component** | **Final Conc.** | **Volume for 20 ml** |
|---|---|---|
| DFNB | | 20 ml |
| bFGF | 20ng/ml | 4µl (1:5,000 dilution from 100µg/ml stock) |
| EGF | 20ng/ml | 1 µl (1:20,000 dilution from 400µg/ml stock) |
| IGF-I | 50ng/ml | 10µl (1:2,000 dilution from 100µg/ml stock) |

## Claims

1. An otic neural progenitor (ONP) cell culture substrate comprising a protein matrix composed of laminin 211, laminin 521 and laminin 111, wherein the amounts of laminin 211, the laminin 521 and the laminin 111 in the otic neural progenitor (ONP) cell culture substrate are defined by a ratio X:Y:Z of (laminin 211):(laminin 521):(laminin 111) in which:
X is from about 1 to about 10;
Y is from about 1 to about 5; and
Z is from about 1 to about 5.

2. The otic neural progenitor (ONP) cell culture substrate of claim 1, wherein
X is from about 2 to about 6;
Y is about 1; and
Z is about 1.

3. The otic neural progenitor (ONP) cell culture substrate of claim 1 or claim 2, wherein the laminin 211, laminin 111 and laminin 521 are human laminin 221, human laminin 111 and human laminin 521.

4. The otic neural progenitor (ONP) cell culture substrate of any one of claims 1-3, wherein the laminin 211, laminin 111 and laminin 521 are recombinant.

5. A method for expanding a population of otic neural progenitor (ONP) cells, the method comprising:
(a) culturing the cells on a first cell culture substrate composed of laminin 211, laminin 111 and laminin 521 for one passage; and subsequently
(b) culturing the cells on a second cell culture substrate composed of laminin 211, laminin 111 and laminin 521 for a further passage, wherein the amount of laminin 211 in the second cell culture substrate is increased relative to the amounts of laminin 111 and laminin 521 in the first cell culture substrate;
optionally
(c) culturing the cells on a third cell culture substrate composed of laminin 211, laminin 111 and laminin 521 wherein the amount of laminin 211 in the third cell culture substrate) is increased relative to the amounts of laminin 111 and laminin 521 in the second cell culture substrate.

6. A method as claimed in claim 5, wherein the amounts of laminin 211, the laminin 111 and the laminin 521 in the cell culture substrate in step (a) are defined by a ratio X:Y:Z of (laminin 211):(laminin 111):(laminin 521) in which
X is from about 1 to about 10;
Y is from about 1 to about 5; and
Z is from about 1 to about 5.

7. A method as claimed in claim 5 or claim 6, wherein the laminin 211, laminin 111 and laminin 521 are:
(i) human laminin 221, human laminin 111 and human laminin 521 and/or
(ii) recombinant.

8. The method of any one of claims 5 to 7, wherein the ONP cells are human cells.

9. The method of any one of claims 5 to 8, wherein the method further comprises a step before step (a) of culturing the ONP cells on a cell culture substrate consisting of laminin 111 and laminin 521 for one passage, optionally wherein the laminin 111 and the laminin 521 are human laminin 111 and human laminin 521.

10. The method of any one of claims 5 to 9, wherein a cell culture medium is added to the cells on the cell culture substrate comprising at least one growth factor selected from the group of an FGF, an EGF and an IGF.

## Patentansprüche

1. ONP(Otic Neural Progenitor)-Zellkultursubstrat, umfassend eine Proteinmatrix, die aus Laminin 211, Laminin 521 und Laminin 111 zusammengesetzt ist, wobei die Mengen an Laminin 211, dem Laminin 521 und dem Laminin 111 in dem ONP(Otic Neural Progenitor)-Zellkultursubstrat durch ein Verhältnis X:Y:Z von (Laminin 211):(Laminin 521):(Laminin 111) definiert sind, in dem:
X für etwa 1 bis etwa 10 steht;
Y für etwa 1 bis etwa 5 steht; und
Z für etwa 1 bis etwa 5 steht.

2. ONP(Otic Neural Progenitor)-Zellkultursubstrat nach Anspruch 1, wobei
X für etwa 2 bis etwa 6 steht;
Y für etwa 1 steht; und
Z für etwa 1 steht.

3. ONP(Otic Neural Progenitor)-Zellkultursubstrat nach Anspruch 1 oder Anspruch 2, wobei es sich bei Laminin 211, Laminin 111 und Laminin 521 um menschliches Laminin 221, menschliches Laminin 111 und menschliches Laminin 521 handelt.

4. ONP(Otic Neural Progenitor)-Zellkultursubstrat nach einem der Ansprüche 1-3, wobei das Laminin 211, das Laminin 111 und das Laminin 521 rekombinant sind.

5. Verfahren zur Expansion einer Population von ONP(Otic Neural Progenitor)-Zellen, wobei das Verfahren Folgendes umfasst:
(a) Kultivieren der Zellen auf einem ersten Zellkultursubstrat, das aus Laminin 211, Laminin 111 und Laminin 521 zusammengesetzt ist, für eine Passage; und anschließend
(b) Kultivieren der Zellen auf einem zweiten Zellkultursubstrat, das aus Laminin 211, Laminin 111 und Laminin 521 zusammengesetzt ist, für eine weitere Passage, wobei die Menge an Laminin 211 im zweiten Zellkultursubstrat in Bezug auf die Mengen an Laminin 111 und Laminin 521 im ersten Zellkultursubstrat erhöht ist; gegebenenfalls
(c) Kultivieren der Zellen auf einem dritten Zellkultursubstrat, das aus Laminin 211, Laminin 111 und Laminin 521 zusammengesetzt ist, wobei die Menge an Laminin 211 im dritten Zellkultursubstrat) in Bezug auf die Mengen an Laminin 111 und Laminin 521 im zweiten Zellkultursubstrat erhöht ist.

6. Verfahren gemäß Anspruch 5, wobei die Mengen an Laminin 211, dem Laminin 111 und dem Laminin 521 in dem Zellkultursubstrat in Schritt (a) durch ein Verhältnis X:Y:Z von (Laminin 211):(Laminin 111):(Laminin 521) definiert sind, in dem
X für etwa 1 bis etwa 10 steht;
Y für etwa 1 bis etwa 5 steht; und
Z für etwa 1 bis etwa 5 steht.

7. Verfahren gemäß Anspruch 5 oder Anspruch 6, wobei das Laminin 211, das Laminin 111 und das Laminin 521:
(i) humanes Laminin 221, humanes Laminin 111 und humanes Laminin 521 sind und/oder
(ii) rekombinant sind.

8. Verfahren nach einem der Ansprüche 5 bis 7, wobei es sich bei den ONP-Zellen um menschliche Zellen handelt.

9. Verfahren nach einem der Ansprüche 5 bis 8, wobei das Verfahren ferner einen Schritt vor Schritt (a) umfasst, bei dem die ONP-Zellen auf einem Zellkultursubstrat, das aus Laminin 111 und Laminin 521 besteht, für eine Passage kultiviert werden, gegebenenfalls wobei es sich bei dem Laminin 111 und dem Laminin 521 um menschliches Laminin 111 und menschliches Laminin 521 handelt.

10. Verfahren nach einem der Ansprüche 5 bis 9, wobei zu den Zellen auf dem Zellkultursubstrat ein Zellkulturmedium gegeben wird, das mindestens einen Wachstumsfaktor umfasst, der aus der Gruppe eines FGF, eines EGF und eines IGF ausgewählt ist.

## Revendications

1. Substrat de culture de cellules de progéniteur neuronal otique (ONP) comprenant une matrice protéique composée de laminine 211, laminine 521 et laminine 111, dans lequel les quantités de laminine 211, de laminine 521 et de laminine 111 dans le substrat de culture de cellules de progéniteur neuronal otique (ONP) sont définies par un rapport X:Y:Z de (laminine 211):(laminine 521):(laminine 111) dans lequel :
X est d'environ 1 à environ 10 ;
Y est d'environ 1 à environ 5 ; et
Z est d'environ 1 à environ 5.

2. Substrat de culture de cellules de progéniteur neuronal otique (ONP) selon la revendication 1, dans lequel
X est d'environ 2 à environ 6 ;
Y est d'environ 1 ; et
Z est d'environ 1.

3. Substrat de culture de cellules de progéniteur neuronal otique (ONP) selon la revendication 1 ou la revendication 2, dans lequel la laminine 211, la laminine 111 et la laminine 521 sont la laminine 221 humaine, la laminine 111 humaine et la laminine 521 humaine.

4. Substrat de culture de cellules de progéniteur neuronal otique (ONP) selon l'une quelconque des revendications 1 à 3, dans lequel la laminine 211, la laminine 111 et la laminine 521 sont recombinantes.

5. Procédé d'expansion d'une population de cellules progénitrices neuronales otiques (ONP), le procédé comprenant :
(a) la culture des cellules sur un premier substrat de culture de cellules composé de laminine 211, de laminine 111 et de laminine 521 pour un passage ; et ensuite
(b) la culture des cellules sur un second substrat de culture de cellules composé de laminine 211, de laminine 111 et de laminine 521 pour un passage supplémentaire, la quantité de laminine 211 dans le second substrat de culture de cellules étant augmentée par rapport aux quantités de laminine 111 et de laminine 521 dans le premier substrat de culture cellulaire ; éventuellement
(c) la culture des cellules sur un troisième substrat de culture de cellules composé de laminine 211, de laminine 111 et de laminine 521, la quantité de laminine 211 dans le troisième substrat de culture de cellules étant augmentée par rapport aux quantités de laminine 111 et de laminine 521 dans le second substrat de culture de cellules.

6. Procédé selon la revendication 5, dans lequel les quantités de laminine 211, de laminine 111 et de laminine 521 dans le substrat de culture de cellules dans l'étape (a) sont définies par un rapport X:Y:Z de (laminine 211):(laminine 111):(laminine 521) dans lequel
X est d'environ 1 à environ 10 ;
Y est d'environ 1 à environ 5 ; et
Z est d'environ 1 à environ 5.

7. Procédé selon la revendication 5 ou la revendication 6, dans lequel la laminine 211, la laminine 111 et la laminine 521 sont :
(i) la laminine 221 humaine, la laminine 111 humaine et la laminine 521 humaine et/ou
(ii) recombinantes.

8. Procédé selon l'une quelconque des revendications 5 à 7, dans lequel les cellules ONP sont des cellules humaines.

9. Procédé selon l'une quelconque des revendications 5 à 8, dans lequel le procédé comprend en outre une étape avant l'étape (a) de culture des cellules ONP sur un substrat de culture de cellules constitué de laminine 111 et de laminine 521 pour un passage, facultativement dans lequel la laminine 111 et la laminine 521 sont la laminine humaine 111 et la laminine humaine 521.

10. Procédé selon l'une quelconque des revendications 5 à 9, dans lequel un milieu de culture de cellules est ajouté aux cellules sur le substrat de culture de cellules comprenant au moins un facteur de croissance choisi dans le groupe d'un FGF, d'un EGF et d'un IGF.
